(19) Europäisches Patentamt
European Patent Office
Office européen des brevets

(11) Veröffentlichungsnummer : **0 030 333**
**B1**

(12) # EUROPÄISCHE PATENTSCHRIFT

(45) Veröffentlichungstag der Patentschrift : **09.03.83**

(51) Int. Cl.³ : **C 07 C143/825**, C 08 J 9/10

(21) Anmeldenummer : **80107422.0**

(22) Anmeldetag : **27.11.80**

(54) **Neue Alkyldiphenylether-sulfonsäurehydrazide, ihre Herstellung und Verwendung als Treibmittel.**

(30) Priorität : **06.12.79 DE 2949069**

(43) Veröffentlichungstag der Anmeldung : **17.06.81 Patentblatt 81/24**

(45) Bekanntmachung des Hinweises auf die Patenterteilung : **09.03.83 Patentblatt 83/10**

(84) Benannte Vertragsstaaten : **BE DE FR GB IT NL**

(56) Entgegenhaltungen :
**CH A 598 301**
**DE A 1 494 975**
**DE C 821 423**
**GB A 1 123 823**
**US A 3 925 466**

(73) Patentinhaber : **BAYER AG**
**Zentralbereich Patente, Marken und Lizenzen**
**D-5090 Leverkusen 1, Bayerwerk (DE)**

(72) Erfinder : **Horstmann, Walter, Dr.**
**Eichenweg 17**
**D-5060 Bergisch-Gladbach 2 (DE)**
Erfinder : **Hammerström, Knut, Dr.**
**Pehlengarten 3**
**D-5060 Bergisch-Gladbach 1 (DE)**

**EP 0 030 333 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Neue Alkyldiphenylether-sulfonsäurehydrazide, ihre Herstellung und Verwendung als Treibmittel

Die Erfindung betrifft neue Alkyldiphenylether-sulfonsäurehydrazide, ein Verfahren zu ihrer Herstellung sowie ihre Verwendung als Treibmittel zur Herstellung von porösen Kautschuken und Schaumstoffen aus thermoplastischen Materialien.

Aus der US-A 2 552 065 ist 4,4'-Bis-(hydrazino-sulfonyl-phenyl)-ether und seine Verwendung als Treibmittel zur Herstellung feinporiger Kautschuke und Kunststoffe bekannt.

Es wurden neue Bis- und Tris-sulfonsäurehydrazide von gegebenenfalls halogenierten Mono- und Dialkyl-diphenylethern der Formel

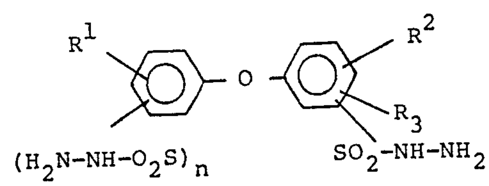

(I)

worin

$R_1$ und $R_2$ gleich oder verschieden sind und für Halogen oder einen niederen Alkylrest stehen, und $R_1$ oder $R_2$ Wasserstoff bedeuten können,

$R_3$ einen niederen Alkylrest bedeutet und

n für die Zahlen 1 oder 2 steht,

gefunden.

Niedere Alkylreste können erfindungsgemäß geradkettig oder verzweigte Kohlenwasserstoffreste mit bis zu etwa 4 Kohlenstoffatomen sein. Beispielsweise seien genannt : Methyl, Ethyl, Propyl, Isopropyl, Butyl, Isobutyl und tertiär Butyl. Als bevorzugter niederer Alkylrest sei der Methylrest genannt.

Halogen kann erfindungsgemäß Fluor, Chlor, Brom oder Jod, bevorzugt Chlor oder Brom sein.

Eine bevorzugte Gruppe von Sulfonsäurehydraziden im Rahmen der Formel (I) sind Verbindungen der Formel

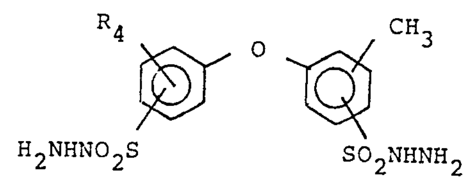

(II)

worin

$R_4$ für Wasserstoff, Chlor oder Methyl und

n für die Zahlen 1 oder 2 steht.

Es wurde außerdem ein Verfahren zur Herstellung der neuen Alkyldiphenylether-sulfonsäurehydrazide gefunden, das dadurch gekennzeichnet ist, daß man Alkyldiphenylether, gegebenenfalls in Gegenwart eines Säurechlorids, im Temperaturbereich von 0 bis 120 °C mit Chlorsulfonsäure zu Alkyldiphenyletherdi- oder -trisulfochlorid chlorsulfoniert und dieses anschließend in einem Lösungsmittel oder Lösungsmittelgemisch im Temperaturbereich von 0 bis 100 °C mit Hydrazin umsetzt.

Das erfindungsgemäße Verfahren kann anhand der folgenden Reaktionsgleichungen erläutert werden :

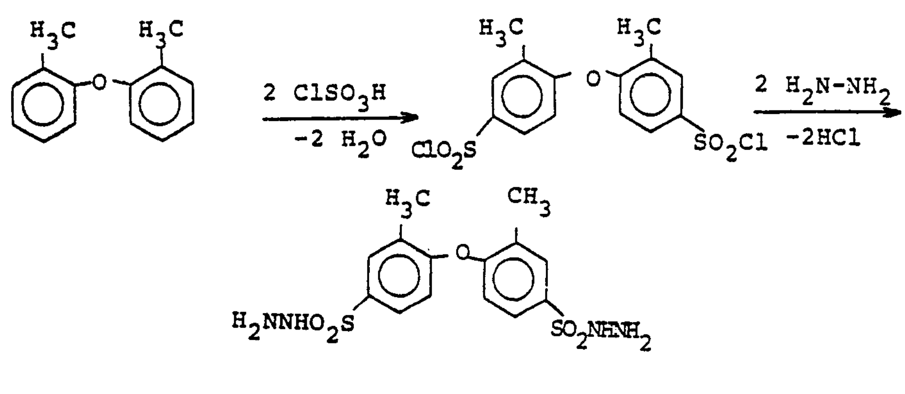


Alkyl-diphenylether für das erfindungsgemäße Verfahren können Verbindungen der Formel

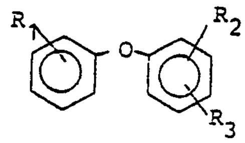

(III)

worin $R_1$, $R_2$ und $R_3$ die oben genannte Bedeutung haben, sein.

Die als Ausgangsprodukte verwendeten Monoalkyl-diphenylether lassen sich durch Umsetzung von Halogenbenzolen mit Alkylphenolen oder von Phenol mit Alkyl-halogenbenzolen herstellen (JA 104 672). Beispielsweise bildet sich aus Brombenzol und o-Kresol bzw. aus Phenol und o-Chlortoluol 2-Methyl-diphenylether. Die verwendeten Dialkyl-diphenylether werden analog aus Alkylphenolen und Alkylhalogen-benzolen hergestellt, z. B. wird 2,2'-Dimethyl-diphenylether aus o-Kresol und o-Chlortoluol gewonnen. Isomerengemische von Dimethyldiphenylethern (Ditolylethern) fallen auch bei der technischen Kresolsynthese aus Chlortoluolen als Nebenprodukte an (Industrial and Engineering Chemistry, Volume *38*, 254 (1946). Diese Gemische können entweder direkt oder nach destillativer Auftrennung in die reinen Ditolyletherisomeren in das erfindungsgemäße Verfahren eingesetzt werden. Alkyl-chlor-diphenylether erhält man beispielsweise aus der Umsetzung von Dichlorbenzolen mit Kresolen in Gegenwart von Kupferkatalysatoren.

Folgende Alkyl-diphenylether können beispielsweise zum Einsatz kommen : 2-Methyl-diphenylether, 2-Methyl-3'-chlordiphenylether, 3-Methyl-3'-chlordiphenylether, 2-Ethyl-diphenylether, 2,2'-, 2,3'-, 2,4'-, 3,3'-, 3,4'- und 4,4'-Ditolylether, 2,2'-Diethyldiphenylether, 3-Methyl-6-chlor-diphenylether, 2,5-Dimethyl-diphenylether, 2,6-Dimethyl-diphenylether und 3-Methyl-6-brom-diphenylether.

Die Verwendung von Chlorsulfonsäure für das erfindungsgemäße Verfahren ist an sich bekannt (Am. Soc. *53*, 1 112). Zur vollständigen Umsetzung der Alkyldiphenylether arbeitet man in der Regel mit überschüssiger Chlorsulfonsäure. Pro Mol Alkyldiphenylether kommen so im allgemeinen 6 bis 12 Mol Chlorsulfonsäure zur Anwendung.

Zur Vermeidung eines größeren Chlorsulfonsäure-Überschusses hat es sich als vorteilhaft erwiesen, die Umsetzung unter Zugabe von anorganischen Säurechloriden, insbesondere von Thionylchlorid durchzuführen.

Die Chlorsulfonierung der Alkyldiphenylether wird im Temperaturbereich von 0 bis 120 °C, vorzugsweise von 30 bis 80 °C bei Normaldruck ausgeführt. Im allgemeinen arbeitet man ohne Lösungs- oder Verdünnungsmittel. Es ist jedoch auch möglich, die Umsetzung in Lösungsmitteln wie Methylenchlorid, Chloroform oder Tetrachlorethan durchzuführen.

In einer anderen Ausführungsform des erfindungsgemäßen Verfahrens werden die gegebenenfalls halogenierten Mono-oder Dialkyldiphenylether der Formel (III) durch Sulfierung mit gegebenenfalls Schwefeltrioxid enthaltender Schwefelsäure zunächst in Alkyl-diphenylether-di- oder trisulfonsäuren überführt und diese in Form der Alkalisalze mit einem anorganischen Säurechlorid, wie z. B.

Phosphortri- oder -pentachlorid oder Thionylchlorid in die entsprechenden Sulfonsäurechloride überführt.

Zur Sulfierung kann man Schwefelsäure mit einer Konzentration über 90 Gew.-%, vorzugsweise 96 bis 100 Gew.-%, verwenden und im Temperaturbereich von Null bis 150 °C, bevorzugt bei 40 bis 120 °C arbeiten. Die Verwendung von Oleum mit Gehalten bis zu 65 Gew.-% Schwefeltrioxid ist ebenfalls möglich.

Die so hergestellten Di- und Trisulfonsäurechloride der Alkyl-diphenylether werden erfindungsgemäß in einem Lösungsmittel mit Hydrazin zu den entsprechenden Di- und Trisulfonsäurehydraziden umgesetzt. Als Lösungsmittel seien Wasser, niedere Alkohole, wie Methanol, Ethanol und Isopropanol, Methylenchlorid, Dichlorethan, Cyclohexan, Toluol, Chlorbenzol und Gemische dieser Lösungsmittel genannt.

Das Hydrazin für das erfindungsgemäße Verfahren wird im allgemeinen in Form des Hydrats eingesetzt.

Das Hydrazin wird beim erfindungsgemäßen Verfahren im allgemeinen im Überschuß eingesetzt. Bevorzugt wird ein Hydrazin-Überschuß von 1,2 bis 1,7 Mol, bezogen auf 1 Mol Alkyldiphenylethersulfochlorid eingesetzt.

Die Umsetzung der Alkyl-diphenylether-sulfonsäurechloride mit Hydrazin erfolgt im allgemeinen im Temperaturbereich von 0 bis 100 °C, bevorzugt von 10 bis 50 °C und bei normalem Druck ; sie kann aber auch bei Unter- oder Überdruck durchgeführt werden.

Es ist vorteilhaft, die Umsetzung der Alkyl-diphenylether-sulfonsäurechloride in Gegenwart einer an organischen Base auszuführen, um die bei der Umsetzung freiwerdende Salzsäure zu binden und Hydrazinhydrat einzusparen.

Das erfindungsgemäße Verfahren kann beispielsweise wie folgt durchgeführt werden :

Die Alkyl-diphenylether werden in einer ersten Reaktionsstufe mit Chlorsulfonsäure und einem Säurechlorid (in der Regel Thionylchlorid) chlorsulfiert, das Reaktionsgemisch in Eiswasser ausgetragen, die kristallin anfallenden Bis- oder Trissulfonsäurechloride isoliert und säurefrei gewaschen.

In einer zweiten Reaktionsstufe wird Hydrazinhydrat in einem geeigneten Lösungsmittel oder Lösungsmittelgemisch vorgelegt und das Bis- oder Trissulfonsäurechlorid anteilweise zugegeben.

Vorteilhafterweise hält man das Reaktionsgemisch durch Zutropfen einer anorganischen Base bei einem pH-Wert von 7 bis 11. Nach Beendigung der Umsetzung säuert man gegebenenfalls mit einer Mineralsäure bis pH 5 bis 6 an und saugt das ausgefallene Alkyl-diphenylether-sulfonsäurehydrazid ab. Wegen der Zersetzlichkeit dieser Bis- und Trissulfonsäurehydrazide trocknet man zweckmäßig bei Temperaturen von 30 bis 40 °C im Vakuum.

Die erfindungsgemäßen neuen Alkyl-diphenylether-sulfonsäurehydrazide können als Treibmittel zur Herstellung von Schwamm-, Moos- und Zellkautschuken aus natürlichem und synthetischem Kautschuk sowie von Schaumstoffen aus thermoplastischem Materialien und von Elastomeren verwendet werden. Die erfindungsgemäß zu Schäumen verarbeiteten Kunststoffe werden in der Regel durch Polymerisation hergestellt. Beispielsweise seien folgende Kunststoffe genannt : Polyvinylchlorid, Polyethylen, Polypropylen, Copolymerisate aus Vinylchlorid/Vinylacetat, Ethylen/Vinylacetat und Propylen/Vinylacetat, Polystyrol, Acrylnitril-Butadien-Styrol-Polymere (ABS), Produkte auf Basis von Celluloseestern, Mischungen aus Polyvinylchlorid und ABS-Polymeren bzw. Acrylnitril-Butadien-Copolymeren.

Geeignete Kautschuke für die Herstellung von zelligen bzw. porösen Artikeln unter Verwendung der erfindungsgemäßen Treibmittel sind z. B. Naturkautschuk oder Synthesekautschuke. Die synthetischen Polymeren werden z. B. ausgehend von konjugierten Diolefinen wie Butadien, Chlorbutadien, Dimethylbutadien, Isopren und seinen Homologen erhalten oder stellen Copolymerisate derartig konjugierter Diolefine mit polymerisierbaren Vinylverbindungen wie Styrol, Alpha-Methylstyrol, Acrylnitril, Methacrylnitril oder Acrylaten dar. Weiterhin sind als Synthesekautschuke geeignet : Ethylen-Vinylacetat-Copolymerisate, Ethylen-Propylen-Dien-Terpolymerisate, z. B. mit Dienen wie 5-Ethylidennorbornen oder Dicyclopentadien als Terkomponente oder Verschnitte der genannten Polymeren.

Bevorzugt werden die erfindungsgemäßen Treibmittel zur Herstellung poröser Gummiartikel verwendet.

Die erfindungsgemäßen Treibmittel werden im allgemeinen in Mengen von 0,01 bis 40 Gew.-%, bevorzugt in Mengen von 0,1 bis 30 Gew.-%, bezogen auf den Kunststoff, zugegeben. Es ist möglich, die erfindungsgemäßen Treibmittel auch in Kombination mit bekannten chemischen Treibmitteln, wie z. B. Azodicarbonamid, zu verwenden.

Zur Herstellung der porösen Kunststoffartikel werden die Treibmittel-Kunststoffmischungen auf Temperaturen erhitzt, bei denen die Zersetzung der Treibmittel unter Aufschäumen des Kunststoffes erfolgt. Diese Temperaturen liegen für die erfindungsgemäßen Treibmittel im Bereich von 100 bis 250 °C, vorzugsweise bei 120 bis 180 °C.

Die Zugabe der erfindungsgemäßen Treibmittel in die zu verschäumenden Mischungen kann auf übliche Weise erfolgen, beispielsweise auf Mischwalzwerken oder Innenmischern. Sie können gleichzeitig mit der Zugabe anderer Mischungsbestandteile und Hilfsstoffen zugesetzt werden. Solche Hilfsstoffe können beispielsweise sein : aktive oder inaktive Füllstoffe wie Ruß, Silikate, Kreide, Kaolin, Antioxidantien, Wachse, Farbpigmente, Zinkoxid, Fettsäuren und fettsaure Salze wie Stearinsäure und Zinkstearat, Mineralöle, Weichmacher wie z. B. Dioctylphthalat, Butylbenzylphthalat, Trikresylphosphat, Flammschutzmittel wie Chlorparaffin, Gleitmittel und Vulkanisationsbeschleuniger wie z. B. Dithiocarbamate.

Als Treibmittel bei der Herstellung von porösen Kunststoffen werden die folgenden Mono- und Dialkyl-diphenylether-bis- bzw. trissulfonsäurehydrazide bevorzugt eingesetzt :

2-Methyl-diphenylether-4,4'-disulfonsäurehydrazid,
2-Methyl-3'-chlor-diphenylether-4,4'-disulfonsäurehydrazid,
3-Methyl-3'-chlor-diphenylether-4,4'-disulfonsäurehydrazid,
3-Methyl-6-chlor-diphenylether-4,4'-disulfonsäurehydrazid,
3-Methyl-6-brom-diphenylether-4,4'-disulfonsäurehydrazid,
2,5-Dimethyl-diphenylether-4,4'-disulfonsäurehydrazid,
2,6-Dimethyl-diphenylether-4,4'-disulfonsäurehydrazid,
2,2'-Dimethyl-diphenylether-4,4'-disulfonsäurehydrazid,
2,3'-Dimethyl-diphenylether-4,4'-disulfonsäurehydrazid,
3,3'-Dimethyl-diphenylether-4,4'-disulfonsäurehydrazid,
4,4'-Dimethyl-diphenylether-2,2'-disulfonsäurehydrazid,
2,3'-Dimethyl-diphenylether-4,4',6'-trisulfonsäurehydrazid,
3,4'-Dimethyl-diphenylether-4,6,2'-trisulfonsäurehydrazid.

Im Vergleich zu dem aus der US-A 2 552 065 bekannten Treibmittel Diphenylether-4,4'-disulfonsäurehydrazid haben die erfindungsgemäßen Alkyldiphenylethersulfonsäurehydrazide die folgenden überraschenden Vorteile : bessere präparative und technische Herstellungsmöglichkeit, z. B. liefert 2,2'-Dimethyldiphenylether in quantitativer Ausbeute ein 4,4'-Disulfochlorid, das ohne weitere Reinigung ebenfalls in hoher Ausbeute und Reinheit zu 2,2'-Dimethyldiphenylether-4,4'-disulfonsäurehydrazid umgesetzt werden kann. Die erfindungsgemäßen Trisulfonsäurehydrazide liefern bei Einsatz gleicher Molmengen eine größere Stickstoffgasausbeute. Die Treibwirkung wird durch die Stickstoffentwicklung

bewirkt. Der Vorteil zeigt sich beim Vergleich von Diphenylether-4,4'-disulfohydrazid mit 2,2'-Dimethyldiphenylether-4,4'-disulfohydrazid z. B. darin, daß das letztere eine PVC-Schaum von geringerem spezifischen Gewicht liefert.

Die mit Hilfe der erfindungsgemäßen Treibmittel hergestellten Schaumstoffe können in üblicher Weise als Schaumkunstleder in der Polster-, Täschner- und Schuhindustrie, in geschäumten Bodenbelegen, in Schwimmkörpern aller Art, in Kabelummantelungen, in geschäumten Folien für Dekorations- und Verpackungszwecke, in geschäumten Extrusionsartikeln, wie Rohre und Profile aller Art, in geschäumten Spritzgießteilen für die Rundfunk-, Möbel- und Phonoindustrie sowie in gesinterten Rotationsgußteilen eingesetzt werden.

Beispiel 1

198 g (1,0 Mol) 2,2'-Ditolylether werden innerhalb von 1 bis 2 Stunden in 933 g (8,0 Mol) Chlorsulfonsäure eingetropft, so daß sich die Innentemperatur bei 50 °C hält. Man rührt 1 Stunde nach und tropft dann innerhalb von 2 bis 3 Stunden bei 50 ± 2 °C 262 g (2,2 Mol) Thionylchlorid ein. Nach Beendigung der Gasentwicklung kühlt man auf Raumtemperatur herunter und trägt langsam auf 4 000 g Wasser aus, so daß die Temperatur 25 °C nicht übersteigt. Man rührt einige Stunden nach, bis vollständige Kristallisation eingetreten ist, saugt das Reaktionsprodukt dann ab und wäscht mineralsäurefrei. Nach dem Trocknen bei 50 °C erhält man 387 g 2,2'-Ditolylether-4,4'-disulfochlorid. Schmelzpunkt 121 bis 123 °C (aus Xylol-Petrolether 2/1).

In einem 3 1 Vierhalskolben mit Rührer, Thermometer, Tropftrichter und pH-Elektrode legt man eine Lösung von 150 g Hydrazinhydrat (3,0 Mol) in 600 g Wasser vor und trägt bei 20 bis 25 °C im Verlaufe von 1 Stunde 395 g (1 Mol) entsprechend 670 g wasserfeuchtes 2,2'-Ditolylether-4,4'-disulfochlorid ein. Durch Zutropfen von 50 %iger Natronlauge hält man den pH-Wert bei 9 bis 9,5. Man rührt unter pH-Konstanthaltung 1 Stunde nach, säuert mit ca. 100 g halbkonzentrierter Schwefelsäure bis pH 5,5 an, isoliert das Reaktionsprodukt und wäscht mit Wasser chloridfrei. Nach dem Trocknen im Vakuum bei 30 bis 35 °C erhält man 350 g 2,2'-Ditolylether-4,4'-disulfonsäurehydrazid der Formel

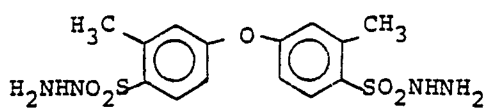

entsprechend 90 bis 91 % der Theorie, bezogen auf 2,2'-Ditolylether. Schmelzpunkt (aus Ethanol/Wasser) : 175 °C unter Zersetzung.

Beispiel 2

39,6 g (0,2 Mol) 3,4'-Ditolylether werden, wie im Beispiel 1 beschrieben, mit Chlorsulfonsäure und Thionylchlorid umgesetzt. Das Reaktionsgemisch wird bei 0-5 °C auf Eiswasser ausgetragen, das ausgefällte Reaktionsprodukt isoliert und säurefrei gewaschen. Nach dem Trocknen im Vakuum bei 40 °C erhält man 66 g eines Trisulfochloridgemisches, das nach Auflösung in Toluol und Wiederausfällen mit Petrolether einen Schmelzpunkt von 143-146 °C zeigt. Schwefelgehalt 19,3 % (Theorie 19,5 %). Laut Kernresonanz-Spektrum besteht das Reaktionsprodukt zu ca. 80 % aus 3,4' -Ditolylether-2',4,6-trisulfonsäurechlorid der Formel

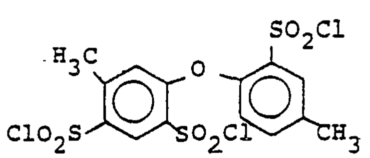

Die restlichen ca. 20 % stellen Isomere dieses Trissulfochlorids dar. Zur Überführung ins Trissulfohydrazidgemisch (analog Beispiel 1) setzt man 49,4 g (0,1 Mol) des obigen Trissulfochlorids mit einer Lösung von 22,5 g Hydrazinhydrat (0,45 Mol) in 200 g Wasser um. Nach dem Trocknen im Vakuum bei 35 °C erhält man 32,8 g eines Gemisches von 3,4'-Ditolylether-trissulfohydraziden. Eine Reinigung kann durch Auflösen in verdünnter Natronlauge und Wiederausfällen mit verdünnter Mineralsäure erfolgen. Schmelzpunkt 148 °C unter Zersetzung.

Beispiel 3

In 120 g konzentrierter Schwefelsäure werden, bei Raumtemperatur beginnend, 59,4 g (0,3 Mol) 4,4'-Ditolylether eingetragen. Dabei steigt die Innentemperatur auf ca. 70 °C an. Man heizt auf 110 bis 115 °C an, hält diese Temperatur 45 Minuten lang, kühlt auf 50 °C ab und trägt auf 300 g Eis aus. Man fällt

die Disulfonsäure durch Zugabe von 500 ml gesättigter Kochsalzlösung aus, saugt ab, schlämmt den Rückstand in 300 ml gesättigter Kochsalzlösung an, neutralisiert die Suspension mit konzentrierter Natronlauge, saugt wiederum ab und trocknet bei 50 °C im Vakuum. Man erhält 129,3 g eines rohen Dinatriumsalzes der 4,4'-Ditolylether-2,2'-disulfonsäure (Verhältnis Kohlenstoff zu Schwefel 2,71 ; Theorie 2,62).

Zur Herstellung des Disulfochlorids trägt man 40 g des rohen Dinatriumsalzes bei Raumtemperatur in 200 g Thionylchlorid ein, setzt 1 ml Dimethylformamid hinzu und heizt auf 80 °C an. Man hält diese Temperatur so lange, bis die Gasentwicklung abgeschlossen ist, destilliert dann das überschüssige Thionylchlorid weitgehend im Vakuum ab und trägt auf 200 g Eis aus. Der Niederschlag wird isoliert, neutral gewaschen und getrocknet. Nach dem Umkristallisieren aus Xylol erhält man 30,5 Teile 4,4'-Ditolylether-2,2'-disulfochlorid vom Schmelzpunkt 211 bis 213 °C.

Zur Herstellung des Dihydrazids werden bei Raumtemperatur 19,8 g 4,4'-Ditolylether-2,2'-disulfochlorid in eine Mischung von 12,5 g Hydrazinhydrat und 25 g Ethanol eingetragen und 2 Stunden bei 45 bis 50 °C nachgerührt. Man trägt auf 250 g Eiswasser aus, säuert mit verdünnter Schwefelsäure bis pH-Wert 5,5 an, saugt ab und wäscht mit Wasser chloridfrei. Nach dem Trocknen bei 35 °C im Vakuum erhält man 17,2 g 4,4'-Ditolylether-2,2'-disulfonsäurehydrazid der Formel

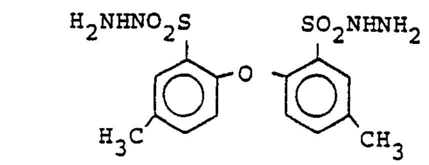

Diese Verbindung hat einen Schmelzpunkt von 172 °C unter Zersetzung.

Beispiel 4

Wie im Beispiel 3 beschrieben, läßt sich in analoger Weise aus 3,3'-Ditolylether 3,3'-Ditolylether-4,4'-disulfonsäurehydrazid der Formel

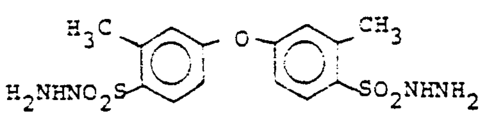

gewinnen. Schmelzpunkt 151 °C (Zersetzung).

Beispiel 5

In einem 0,5 Liter Dreihalskolben mit Rührer, Tropftrichter und Termometer werden 180 g Schwefelsäuremonohydrat auf 50 ± 2 °C erwärmt und bei dieser Temperatur 36,8 g (0,2 Mol) 2-Methyl-diphenylether eingetropft. Man rührt 1 Stunde lang nach, trägt auf 400 g Eiswasser aus, fällt das Reaktionsprodukt durch Zugabe von 400 ml gesättigter Kochsalzlösung aus, neutralisiert wie im Beispiel 3 beschrieben, isoliert und trocknet. Man erhält 54,8 g eines rohen Dinatriumsalzes der 2-Methyl-diphenylether-4,4'-disulfonsäure (Verhältnis Kohlenstoff zu Schwefel 2,49 ; Theorie 2,43).

49 g des rohen Dinatriumsalzes der 2-Methyl-diphenylether-4,4'-disulfonsäure werden nun weiter wie im Beispiel 3 beschrieben mit Thionylchlorid umgesetzt und aufgearbeitet. Man erhält 41,5 g eines Disulfochlorids der Formel

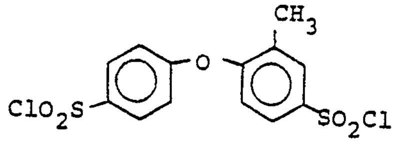

Schmelzpunkt 120-121 °C (aus Toluol).

Die Umsetzung dieses Disulfonsäurechlorids mit Hydrazinhydrat wird ebenfalls wie im Beispiel 3 beschrieben durchgeführt. Aus 38 g Disulfonsäurechlorid erhält man 31,8 g 2-Methyl-diphenylether-4,4'-disulfonsäurehydrazid. Schmelzpunkt 165 °C (Zersetzung). Die Verbindung läßt sich aus wäßrigem

Ethanol umkristallisieren.

## Anwendungsbeispiele

### Beispiel 6

Nachfolgend aufgeführte Mischungsbestandteile wurden auf einem Mischwalzwerk zunächst homogen vermischt :

Tabelle 1

| | Mischung 1 | Mischung 2 |
|---|---|---|
| Naturkautschuk | 70,0 | 70,0 Gew.-Teile |
| Styrolharz | 30,0 | 30,0 Gew.-Teile |
| Zinkoxid | 3,0 | 3,0 Gew.-Teile |
| Kaolin | 10,0 | 10,0 Gew.-Teile |
| Magnesiumcarbonat | 10,0 | 10,0 Gew.-Teile |
| Paraffinwachs | 0,5 | 0,5 Gew.-Teile |
| Stearinsäure | 1,0 | 1,0 Gew.-Teile |
| Farbstoff (Ruß) | 0,2 | 0,2 Gew.-Teile |
| Schwefel | 2,5 | 2,5 Gew.-Teile |
| Dibenzothiazyldisulfid | 1,5 | 1,5 Gew.-Teile |
| 2,2'-Ditolylether-4,4'-disulfonsäurehydrazid | 7,5 | — Gew.-Teile |
| 3,3'-Ditolylether-4,4'-disulfonsäurehydrazid | — | 7,5 Gew.-Teile |

Die fertigen Mischungen wurden zu 10 mm starken Fellen ausgezogen und davon jeweils 430 g so ausgestanzt, daß eine Einlage in einen Formrahmen mit den Abmessungen 200 × 200 × 8 mm (Seitenlänge × Höhe) ermöglicht wurde.

Die Mischungen wurden anschließend — umgrenzt durch die Form — in dampfbeheizten Vulkanisierpressen 12 Minuten bei 140 °C und einem Druck von ca. 100 bar vorvulkanisiert. Nach Ablauf dieser Zeit wurde die Presse geöffnet und die nun vorvulkanisierten Platten expandiert infolge des durch die Treibmittelzersetzung entstandenen Gasdruckes. Die Zellgummiplatten wurden zur endgültigen Ausvulkanisation noch 30 Minuten in Heißluft (drucklos) von 130 °C gelagert. Die so erhaltenen Platten wiesen folgende Werte auf :

Tabelle 2

| | Dichte ($g/cm^3$) | Härte (Shore A) |
|---|---|---|
| Mischung Nr. 1 | 0,19 | 20 |
| Mischung Nr. 2 | 0,21 | 22 |

Die Platten wiesen nach dem Abschälen der Vulkanisationshaut eine gleichmäßige, feine Zellstruktur auf und waren praktisch geruchlos.

### Beispiel 7

Auf einem Mischwalzwerk wurden die nachstehend aufgeführten Mischungsbestandteile homogen miteinander vermischt :

| | |
|---|---|
| Acrylnitril-Butadien-Kautschuk | 70,0 Gew.-Teile |
| Polyvinylchlorid | 30,0 Gew.-Teile |
| Zinkoxid | 5,0 Gew.-Teile |
| Ruß | 35,0 Gew.-Teile |
| Kreide | 25,0 Gew.-Teile |
| Stearinsäure | 1,0 Gew.-Teile |
| Trikresylphosphat | 25,0 Gew.-Teile |
| Alkylsulfonsäureester des Phenols | 12,0 Gew.-Teile |

| | |
|---|---|
| Schwefel | 1,5 Gew.-Teile |
| Diphenylguanidin | 0,9 Gew.-Teile |
| Tetramethylthiurammonosulfid | 0,4 Gew.-Teile |
| Zink-N-dimethyldithiocarbamat | 0,2 Gew.-Teile |
| 2,2'-Ditolylether-4,4'-disulfonsäurehydrazid | 20,0 Gew.-Teile. |

Die fertige Mischung wurde zu einem glatten 5 mm starken Fell ausgezogen und aus diesem wurden 100 × 100 mm große Teststücke ausgestanzt. Diese wurden zwecks Vulkanisation und gleichzeitigem Auftreiben 20 Minuten in Heißluft (drucklos) von 140 °C gelagert. Nach Ablauf dieser Zeit waren die Teststücke ausvulkanisiert und zeigten eine erhebliche Volumenzunahme im Vergleich zu den unvulkanisierten Teststücken. Die Dichte wurde mit 0,13 g/cm³ bestimmt.

Beispiel 8

Ein PVC-Plastisol (eine Anpastung von PVC und Weichmacher) folgender Zusammensetzung :

50,0 g Pasten-PVC, K-Wert 70 (pH des wäßrigen Auszuges = 7,0),
50,0 g Di-2-ethyl-hexyl-phthalat,
0,3 g Diphenylthioharnstoff,
10,0 g 2,2'-Dimethyl-diphenylether-4,4'-disulfonsäurehydrazid

wird in eine gasdichte Stahlform von 100 × 100 × 10 mm Größe eingefüllt und unter der Hochdruckpresse bei 25 bar und 170 °C 10 Minuten verpreßt. Anschließend wird 7 Minuten unter Druck abgekühlt. Der erhaltene Formling wird dann 30 Minuten in Heißluft von 105 °C nachgeheizt und expandiert.

Man erhält einen feinporigen Schaumblock mit einem Raumgewicht von 93 kg/m³. Kernverbrennungen sind nicht zu beobachten.

Beispiel 9

Ein PVC-Plastisol aus :

50,0 g eines verpastbaren Polyvinychlorids vom K-Wert 70 nach DIN 53 726 (Bestimmung der Viskositätszahl und des K-Wertes von Polyvinylchlorid in Lösung) ; pH des wäßrigen Auszuges = 7,0,
38,0 g Di-2-ethyl-hexyl-phthalat,
12,0 g Butyl-benzyl-phthalat,
0,5 g eines handelsüblichen Cadmium/Zink-Stabilisators
2,0 g 2,2'-Dimethyl-diphenylether-4,4'-disulfonsäurehydrazid

wird in einem mit Heißluft von 190 °C beheizten Heißluftkanal, bei einem Auflagegewicht von 850 g/m² und einer Verweilzeit von 1 Minute verschäumt. Man erhält einen feinporigen Schaum mit einem spezifischen Gewicht von 420 kg/m³.

Beispiel 10

Vergleichsweise liefert das Plastisol aus Beispiel 9 mit 2,0 Gew.-Teilen Diphenylether-4,4'-disulfonsäurehydrazid als Treibmittel einen Schaum mit einem spezifischen Gewicht von 500 kg/m³.

**Ansprüche**

1. Di- und Trisulfonsäurehydrazide alkylierter Diphenylether der Formel

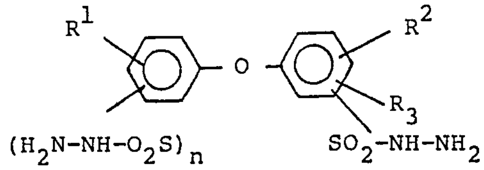

worin

$R_1$ und $R_2$ gleich oder verschieden sind und für Halogen oder einen niederen Alkylrest stehen, und $R_1$ oder $R_2$ Wasserstoff bedeuten können,
$R_3$ einen niederen Alkylrest bedeutet und
n für die Zahlen 1 oder 2 steht.

2. Disulfonsäurehydrazide alkylierter Diphenylether nach Anspruch 1 der Formel

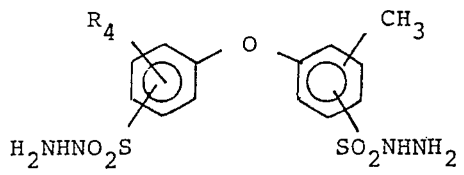

worin $R_4$ für Wasserstoff, Chlor oder Methyl steht.

3. Verfahren zur Herstellung von Alkyldiphenylethersulfonsäurehydraziden, dadurch gekennzeichnet, daß man Alkyl-diphenylether, gegebenenfalls in Gegenwart eines Säurechlorids, im Temperaturbereich von 0 bis 120 °C mit Chlorsulfonsäure zu Alkyldiphenylether, -di oder -trisulfochlorid chlorsulfoniert und diese anschließend in einem Lösungsmittel oder Lösungsmittelgemisch im Temperaturbereich von 0 bis 100 °C mit Hydrazin umsetzt.

4. Verfahren nach Anspruch 3, dadurch gekennzeichnet, daß man die Alkyl-diphenylether-disulfonsäure-chloride durch Sulfierung eines Alkyldiphenylethers mit gegebenenfalls Schwefeltrioxid enthaltender Schwefelsäure im Temperaturbereich von 20 bis 150 °C und nachfolgender Umsetzung mit einem Säurechlorid herstellt.

5. Treibmittel zur Herstellung von porösen Kautschuken, Schaumstoffen aus thermoplastischen Materialien und Elastomeren, enthaltend Alkyldiphenylethersulfonsäurehydrazide der Formel

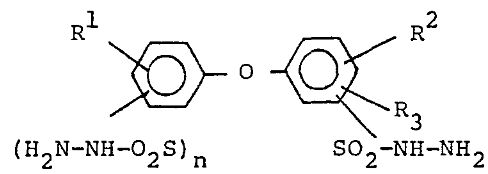

worin

$R_1$ und $R_2$ gleich oder verschieden sind und für Halogen oder einen niederen Alkylrest stehen und $R_1$ oder $R_2$ Wasserstoff bedeuten können,
$R_3$ einen niederen Alkylrest bedeutet und
n für die Zahlen 1 oder 2 steht.

6. Verwendung von Treibmitteln nach Anspruch 5 bei der Herstellung von Schaumstoffen, dadurch gekennzeichnet, daß man Treibmittel mit dem Kunststoff vermischt und die Treibmittel-Kunststoffmischung auf Temperaturen von 100 bis 300 °C erhitzt.

7. Verwendung von Treibmitteln nach den Ansprüchen 5 und 6 zur Herstellung poröser Gummiartikel.

## Claims

1. Di- and tri-sulphonic acid hydrazides of alkylated diphenyl ethers, of the formula

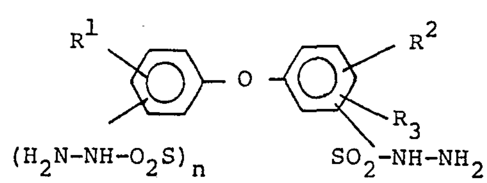

wherein

$R_1$ and $R_2$ are identical or different and represent halogen or a lower alkyl radical and $R_1$ or $R_2$ can denote hydrogen,
$R_3$ denotes a lower alkyl radical and
n represents the number 1 or 2.

2. Disulphonic acid hydrazides of alkylated diphenyl ethers according to Claim 1, of the formula

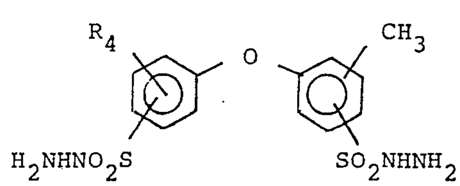

wherein $R_4$ represents hydrogen, chlorine or methyl.

3. Process for the preparation of alkyldiphenyl ether-sulphonic acid hydrazides, characterised in that alkyldiphenyl ethers are chlorosulphonated with chlorosulphonic acid in the temperature range from 0 to 120 °C, if appropriate in the presence of an acid chloride, to give the alkyldiphenyl ether di- or tri-sulphochloride, and this compound is then reacted with hydrazine in a solvent or solvent mixture in the temperature range from 0 to 100 °C.

4. Process according to Claim 3, characterised in that the alkyl-diphenyl ether-disulphonic acid chlorides are prepared by sulphonation of an alkyldiphenyl ether with sulphuric acid, which may contain sulphur trioxide, in the temperature range from 20 to 150 °C and subsequent reaction of the product with an acid chloride.

5. Blowing agents for the production of porous rubbers, foams of thermoplastic materials and elastomers, which contain alkyldiphenyl ether-sulphonic acid hydrazides of the formula

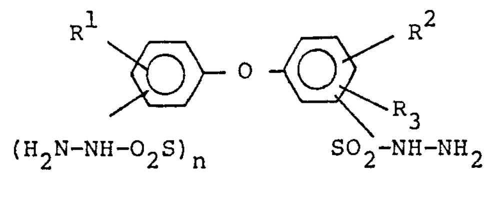

wherein

$R_1$ and $R_2$ are identical or different and represent halogen or a lower alkyl radical and

$R_1$ or $R_2$ can denote hydrogen,

$R_3$ denotes a lower alkyl radical and

n represents the number 1 or 2.

6. Use of blowing agents according to Claim 5 in the production of foams, characterised in that the blowing agent is mixed with the resin and the blowing agent/resin mixture is heated to temperatures of 100 to 300 °C.

7. Use of blowing agents according to Claims 5 and 6 for the production of porous rubber articles.

**Revendications**

1. Hydrazides d'acides di- et trisulfoniques de diphényl-éthers alcoylés de formule

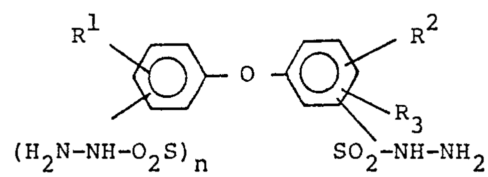

dans laquelle

$R_1$ et $R_2$ sont identiques ou différents et représentent de l'halogène ou un radical alcoyle inférieur et

$R_1$ ou $R_2$ peuvent signifier de l'hydrogène,

$R_3$ représente un radical alcoyle inférieur et

n représente les nombres 1 ou 2.

2. Hydrazides d'acides disulfoniques de diphényl-éthers alcoylés selon la revendication 1 et de formule

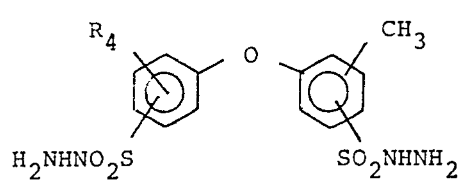

dans laquelle $R_4$ représente de l'hydrogène, du chlore ou un méthyle.

3. Procédé de fabrication d'hydrazides d'acides alcoyldiphényl-éther-sulfoniques, caractérisé en ce qu'on chlorosulfone des alcoyldiphényl-éthers, éventuellement en présence d'un chlorure d'acide, dans l'intervalle de température de 0 à 120 °C avec de l'acide chlorosulfonique pour obtenir 1 ' alcoyldiphényl-éther-di- ou -trisulfochlorure, et en ce qu'on fait ensuite réagir celui-ci dans un solvant ou mélange

solvant dans l'intervalle de température de 0 à 100 °C avec de l'hydrazine.

4. Procédé selon la revendication 3, caractérisé en ce qu'on prépare les chlorures d'acides alcoyldiphényl-éther-disulfoniques par sulfonation d'un alcoyldiphényl-éther avec de l'acide sulfurique contenant éventuellement du trioxyde de soufre dans l'intervalle de température de 20 à 150 °C et par réaction consécutive avec un chlorure d'acide.

5. Agents expanseurs pour la fabrication de caoutchouc, de matières cellulaires en matériaux thermoplastiques et d'élastomères poreux, contenant des hydrazides d'acides alcoyldiphényl-éther-sulfoniques de formule

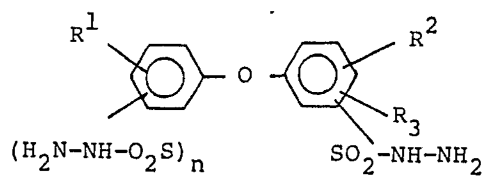

dans laquelle

$R_1$ et $R_2$ sont identiques ou différents et représentent de l'halogène ou un radical alcoyle inférieur et $R_1$ ou $R_2$ peuvent signifier de l'hydrogène,

$R_3$ signifie un radical alcoyle inférieur et

n représente les nombres 1 ou 2.

6. Utilisation des agents expanseurs selon la revendication 5 dans la fabrication de matières cellulaires, caractérisée en ce qu'on mélange l'agent expanseur avec la matière plastique et l'on chauffe le mélange agent expanseur-matière plastique à des températures de 100 à 300 °C.

7. Utilisation d'agents expanseurs selon les revendications 5 et 6 pour la fabrication d'articles poreux en caoutchouc.